# EUROPEAN PATENT APPLICATION

(11) **EP 2 281 565 A2**
(43) Date of publication of application: **09.02.2011**
(21) Application number: 10180966.3
(22) Date of filing: 08.04.2003
(51) Int. Cl.: A61K 31/66, A61P 23/00

(54) **Pharmaceutical compositions containing water-soluble prodrugs of propofol and methods of administering same**

(30) Priority: 08.04.2002 US 370213 P; 08.04.2002 US 370245 P
(62) Divisional of application: 03721545.6
(71) Applicant: Eisai Inc., Woodcliff Lake, NJ 07677 (US)
(72) Inventor: Wingard, Peggy, Houston, TX 77005 (US); Burak, Eric S., East Haddam, CT 06423 (US); Gibiansky, Ekaterina, North Potomac, MD 20878 (US); Vornov, James J., Baltimore, MD 21215 (US)
(74) Representative: HOFFMANN EITLE

(57) **Abstract**

The present invention is directed to pharmaceutical compositions containing water-soluble prodrugs of propofol and methods of administering the prodrug. In one aspect, a method of inducing and/or maintaining a generalized anesthetic state comprises administering by parenteral infusion a prodrug of propofol in an amount sufficient to cause and/or maintain loss of consciousness. In another aspect, a prodrug of propofol is administered for producing a sedated state in a subject.

## Description

### CROSS REFERENCE TO RELATED APPLICATIONS

This application claims benefit under 35 U.S.C. § 119(e) to U.S. Application No. 60/370,213, filed April 8, 2002 and to U.S. Application No. 60/370,245, filed April 8, 2002, the disclosure of each of which is hereby incorporated by reference in its entirety.

### FIELD OF THE INVENTION

The present invention is directed to compositions containing water-soluble prodrugs of propofol and to methods of administering the prodrugs, including methods for inducing and maintaining extended periods of sedation.

### BACKGROUND OF THE INVENTION

Propofol (2,6-diisopropylphenol) is a low molecular weight phenol derivative that is widely used as a hypnotic or sedative agent for intravenous administration in the induction and maintenance of anesthesia or sedation in humans and animals. Among its useful characteristics as an anesthetic drug are: administration via the intravenous route, rapid onset and offset of anesthesia, rapid clearance, and a side-effect profile that makes it preferable to other injectable anesthetics, such as barbiturates.

The use of injectable anesthetic agents generally, and of propofol specifically, in the induction and maintenance of general anesthesia has gained widespread acceptance in anesthetic care over the last 15 years. Intravenous anesthesia with propofol has been described to have several advantages over preexisting methods, such as more readily tolerated induction, since patients need have no fear of masks, suffocation, or the overpowering smell of volatile anesthetics; rapid and predictable recovery; readily adjustable depth of anesthesia by adjusting the IV dose of propofol; a lower incidence of adverse reactions as compared to inhalation anesthetics; and decreased dysphoria, nausea, and vomiting upon recovery from anesthesia [Padfield NL, Introduction, history and development. In: Padfield NL (Ed.) Ed., Total Intravenous Anesthesia. Butterworth Heinemann, Oxford 2000].

In addition to its sedative and anesthetic effects, propofol has a range of other biological and medical applications. For example, it has been reported to be an anti-emetic [McCollum JSC et al., Anesthesia 43 (1988) 239], an anti-epileptic [Chilvers CR, Laurie PS, Anesthesia 45 (1990) 995], and an anti-pruritic [Borgeat et al., Anesthesiology 76 (1992) 510]. Anti-emetic and anti-pruritic effects are typically observed at sub-hypnotic doses, i.e. at doses that achieve propofol plasma concentrations lower than those required for sedation or anesthesia. Antiepileptic activity, on the other hand, is observed over a wider range of plasma concentrations [Borgeat et al., Anesthesiology 80 (1994) 642]. It has further been speculated that propofol, due to its antioxidant properties in biological systems, may be useful in the treatment of inflammatory conditions, especially inflammatory conditions with a respiratory component, and in the treatment of neuronal damage related to neurodegeneration or trauma. Such conditions are believed to be associated with the generation of reactive oxygen species and therefore amenable to treatment with antioxidants [see, e.g. U.S. Patent 6,254,853 to Hendler et al.]

Propofol typically is formulated for clinical use as a oil-in-water emulsion. The formulation has a limited shelf-life and has been shown to be sensitive to bacterial or fungal contamination, which has led to instances of post-surgical infections [Bennett SN et al., N Engl. J Med 333 (1995) 147]. Due to the dense, white color of the formulation, bacterial or fungal contamination cannot be detected by visual inspection of the vial in the first instance.

Not only is propofol poorly water soluble, but it also causes pain at the injection site, which must often be alleviated by using a local anesthetic [Dolin SJ, Drugs and pharmacology. In: N. Padfield, Ed., Total Intravenous Anesthesia. Butterworth Heinemaim, Oxford 2000]. Due to its formulation in a lipid emulsion, its intravenous administration is also associated with undesirable hypertriglyceridemia in patients, especially in patients receiving prolonged infusions [Fulton B and Sorkin EM, Drugs 50 (1995) 636]. Its formulations as a lipid emulsion further makes it difficult to co-administer other IV drugs. Any physical changes to the formulation, such as a change in lipid droplet size, can lead to changes in the pharmacological properties of the drug and cause side effects, such as lung embolisms.

It has further been reported that the use of propofol in anesthesia induction is associated with a significant incidence of apnea, which appears to be dependent on dose, rate of injection, and pre-medication [Reves, JG, Glass, PSA, Lubarsky DA, Non-barbiturate intravenous anesthetics. In: R.D. Miller et al., Eds, Anesthesia. 5th Ed. Churchill Livingstone, Philadelphia, 2000]. Respiratory consequences of administering anesthetic induction doses of propofol, including a reduction in tidal volume and apnea, occur in up to 83% of patients [Bryson et al., Drugs 50 (1995) at 520]. Induction doses of propofol are also known to have a marked hypotensive effect, which is dose- and plasma concentration-dependent [Reves et al., *supra*]*,* The hypotension associated with peak plasma levels after rapid bolus injection.of propofol sometimes requires the use of controlled infusion pumps or the breaking-up of the induction bolus dose into several smaller incremental doses. Further, the short duration of unconsciousness caused by bolus induction doses renders propofol suitable for only brief medical procedures. For all the above reasons, propofol for induction and/or maintenance of anesthesia must normally be administered in an in-patient setting under the supervision of an anesthesiologist, and is often considered inappropriate for use by non-anesthesiologists in an ambulatory or day case setting.

In addition to its use in induction and maintenance of anesthesia, propofol has been used successfully as a sedative to accompany either local or regional anesthesia in conscious patients. Its sedative properties have also been exploited in diagnostic procedures that have an unsettling effect on conscious patients, such as colonoscopy or imaging procedures. Propofol has also been used as a sedative in children undergoing diagnostic imaging procedures or radiotherapy. A recent development is that of patient-controlled sedation with propofol. This technique is preferred by patients and is as effective as anesthesiologist-administered sedation.

Compared with the widely used sedative midazolam or other such agents, propofol provided similar or better sedative effects when the quality of sedation and/or the amount of time that patients were at adequate levels of sedation were measured [see Fulton B and Sorkin EM, Drugs 50 (1995) 636]. The faster recovery and similar or less amnesia associated with propofol makes it an attractive alternative to other sedatives, particularly for patients requiring only short sedation. However, because of the potential for hyperlipidemia associated with the current propofol formulation, and the development of tolerance to its sedative effects, the usefulness of prapofal for patients requiring longer sedation is less well established. For all the reasons given above, there exists a clinical need for aqueous, stable formulations of safe, injectable, or infusible sedative or hypnotic agents.

The development of water soluble and stable prodrugs of propofol, which is described in U.S. Patent 6,204,257 to Stella et al., has made it possible to address these heretofore unmet needs, and to explore the pharmaceutical advantages of an aqueous propofol-pradrug in the induction and maintenance of sedation and anesthesia in patients. The prodrugs of the present invention differ from propofol in that the 1-hydroxy-group of propofol is replaced with a phosphonooxymethyl ether group: 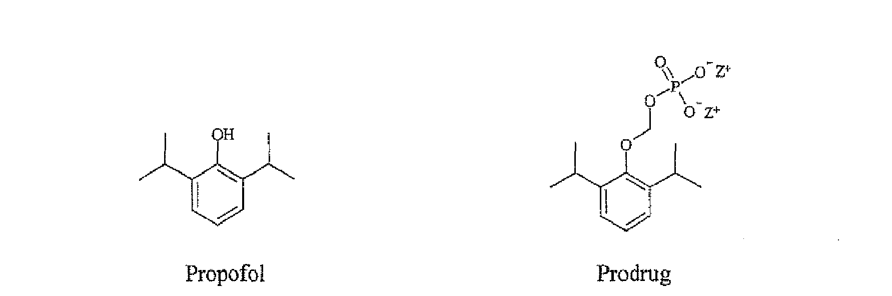 (Z = Hydrogen, alkali metal ion, or amine)
While the present invention is not bound by any theory, the prodrug is believed to undergo hydrolysis by endothelial cell surface alkaline phosphatases to release propofol.

Stella reports that the prodrug has good stability at pH levels suitable for making pharmaceutical formulations, and quickly breaks down *in vivo* under physiological conditions.

### BRIEF SUMMARY OF THE INVENTION

In one aspect, the present invention provides a method of inducing or maintaining a generalized anesthetic state in a subject in need thereof. The method comprises administering at least one bolus injection of a compound of Formula I: 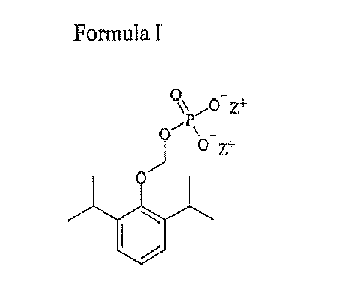 or a pharmaceutically acceptable salt thereof, wherein each Z is independently selected from the group consisting of hydrogen, alkali metal ion, and amine. The compound is administered in an amount of from greater than 10 to about 50 mg per kilogram of body weight.

According to another aspect of the invention, a method of inducing or maintaining a generalized anesthetic state is provided. The method comprises administering an effective amount of a compound of Formula I, or a pharmaceutically acceptable salt thereof, and administering a second anesthetic or sedative agent.

In accordance with another embodiment of the invention, there is provided a method of inducing and maintaining general anesthesia in a subject. The method comprises administering a compound of Formula. I, or a pharmaceutically acceptable salt thereof, in a first amount sufficient to cause loss of consciousness, and administering a compound of Formula I, or a pharmaceutically acceptable salt thereof, in a second amount sufficient to maintain loss of consciousness.

The present invention also includes a method of producing a sedated state in a subject. The method comprises administering to a subject in need thereof a a parenteral bolus injection of a compound of Formula I, or a pharmaceutically acceptable salt thereof, in an amount of from about 2 mg/kg to about 15 mg/kg.

In another embodiment, a method of producing a sedated state in a subject comprises administering to a subject in need thereof a parenteral infusion of a compound of Formula I, or a pharmaceutically acceptable salt thereof, in an amount of from about 5 to about 25 mg/min.

According to another aspect of the present invention, a method of producing a sedated state comprises administering to a subject in need thereof an effective amount of a compound of Formula I, or a pharmaceutically acceptable salt thereof, and administering a second anesthetic or sedative agent.

According to another aspect of the invention, a method of treating at least one condition selected from the group consisting of an epileptic condition, nausea or vomiting, pruritus, pathologic respiratory conditions related to oxidative tissue damage and pathologic conditions having an inflammatory component, comprises administering to a subject in need thereof an effective amount of a compound of Formula I, or a pharmaceutically acceptable salt thereof.

In another aspect, the present invention provides a pharmaceutical composition comprising an anesthetic amount of a compound of Formula I, or a pharmaceutically acceptable salt thereof, a second anesthetic or sedative agent, and a pharmaceutically acceptable carrier, diluent, or excipient.

The present invention also provides a pharmaceutical composition comprising an anti-emetic effective amount of a compound of Formula I, or a pharmaceutically acceptable salt thereof, a second anti-emetic agent, and a pharmaceutically acceptable carrier, diluent, or excipient.

In another aspect, the present invention provides a pharmaceutical composition comprising an anti-pruritic effective amount of a compound of Formula I, or a pharmaceutically acceptable salt thereof, a second anti-pruritic agent, and a pharmaceutically acceptable carrier, diluent, or excipient.

### BRIEF DESCRIPTION OF THE DRAWINGS

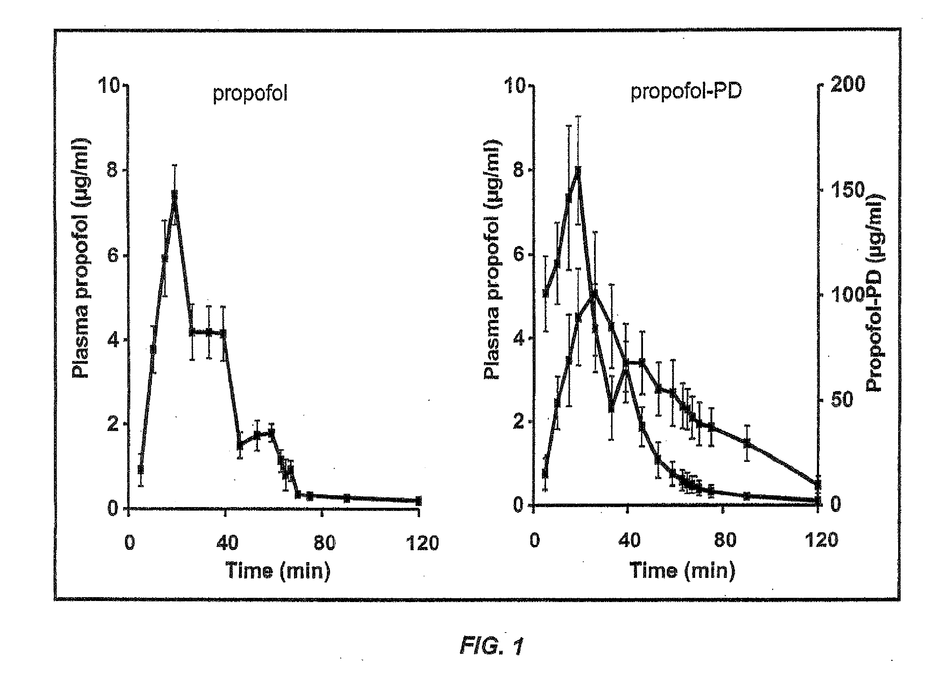

Figure 1 shows the mean measured plasma concentrations of propofol derived from target-controlled infusions of propofol (left panel), and of propofol derived from infusions of O-phosphonooxymethyl propofol disodium salt ("prodrug"; right panel) in human subjects. This study was conducted in nine male volunteers to demonstrate the usefulness of infusing an aqueous solution of C3-phosphonooxymethyl propofol disodium salt in inducing and maintaining a generalized anesthetized state.

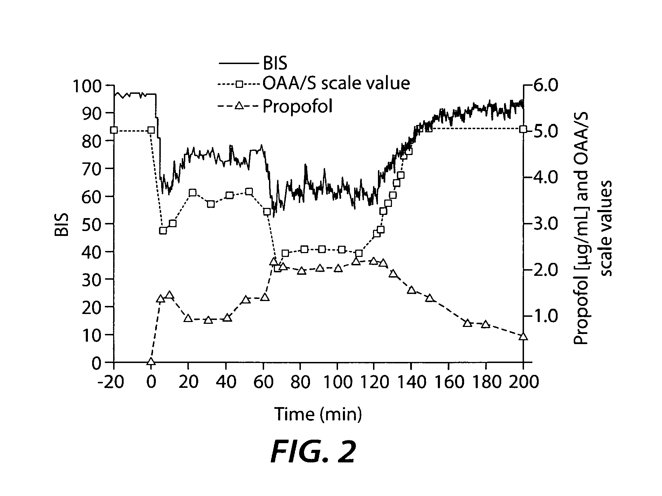

Figure 2 shows the mean results for BIS and OAA/S scale values as well as the measured plasma concentrations of propofal derived from target-controlled infusions of O-phosphonooxymethyl propofol disodium salt in human subjects. This study was conducted in twelve healthy volunteers to demonstrate the usefulness of infusing an aqueous solution of O-phosphonooxymethyl propofol disodium salt in inducing and maintaining a conscious sedated state.

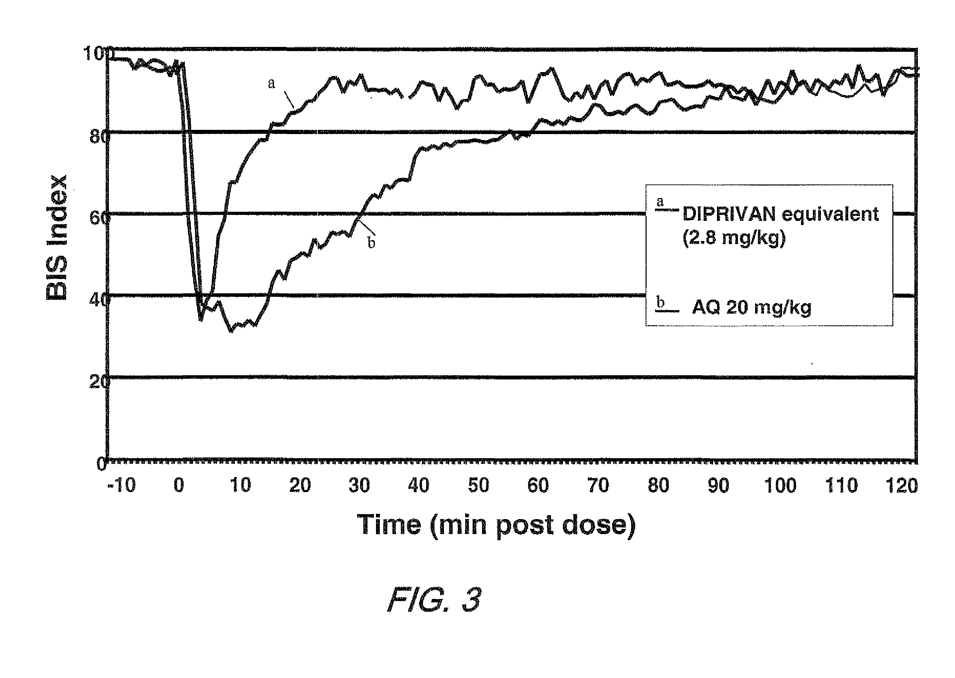

Figure 3 shows the mean alterations in BIS scores of human volunteers dosed with a 20 mg/kg bolus of O-phosphonooxymethyl propofol disodium salt (AQ 20 mg/kg) compared to human volunteers dosed with a rapid infusion of propofol calculated to give an equivalent peak BIS score (DIPRIVAN equivalent, 2.8 mg/kg). The diagram shows that the prodrug can be administered to cause an onset of sedation and anesthesia that is as rapid as that caused by propofol, but that its effect is longer-lasting and shows a more gradual wearing-off.

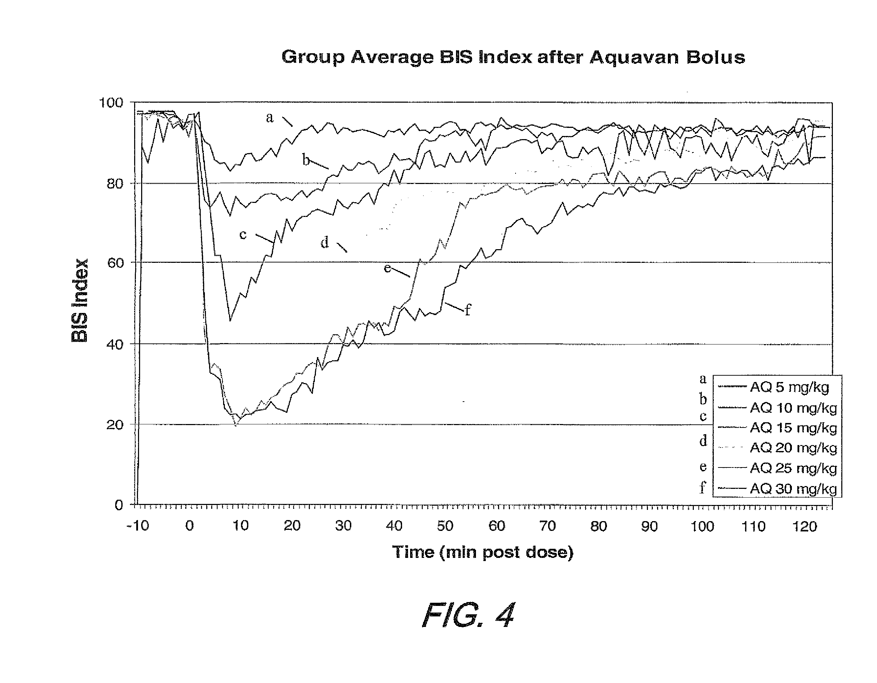

Figure 4 shows the mean alterations of BIS scores of human volunteers dosed width 5 - 30 mg/kg bolus injections of O-phosphonaoxyrnethyl propofol disodium salt. The experimental procedures for this study are those described in EXAMPLE 3.

### DETAILED DESCRIPTION OF THE INVENTION

It has been found that plasma propofol derived from infusions of the propofol prodrug was significantly more potent in suppressing EEG activity and causing a hypnotic effect in human subjects than was plasma propofol derived from infusions of propofol itself (Fig. 3). The excellent and unexpected properties of the prodrug as an infusible agent were further demonstrated by its ability to rapidly induce and maintain a conscious sedated state in human subjects, which could be rapidly adjusted by altering the rate of infusion.

According to one embodiment of the present invention, a generalized anesthetic state is induced or maintained in a subject by administering a parenteral infusion of a prodrug of propofol in an amount sufficient to cause and/or maintain loss of consciousness. The prodrug is a compound of Formula I: 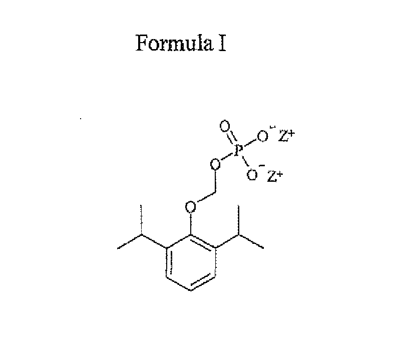 or a pharmaceutically acceptable salt thereof, wherein each Z is independently selected from the group consisting of hydrogen, alkali metal ion, and amine. Each Z preferably is an alkali metal ion, especially a sodium ion.

The compound of Formula I may be administered by itself or may be co-administered together with one or more additional active agents, such as, for example, hypnotic, analgesic, anti-inflammatory, amnesic, muscle relaxant, and sedative agents. Non-limiting examples of such additional agents include midazolam, fentanyl, meperidine, and propofol. Such additional active agents may be incorporated into a pharmaceutical composition containing the compound of Formula I, or may be administered in a separate pharmaceutical formulation. A composition may include a compound of Formula I and one or more additional anesthetic or sedative agents, non-limiting examples of which include midazolam, fentanyl, meperidine, and propofol.

When the compound of Formula I is administered to a subject by parenteral infusion for maintaining general anesthesia, suitable dosages typically range from about 10 mg/min to about 35 mg/min, more typically from about 15 mg/min to about 30 mg/min, and even more typically from about 15 mg/mm to about 20 mg/min.

In another embodiment of the present invention, a conscious sedated state is induced, or maintained over an extended period of time, in a subject. The sedated state can be induced or maintained by administering an effective amount of a compound of Formula I or a pharmaceutically acceptable salt thereof. Any suitable route of administration may be used in the practice of this embodiment, as will be appreciated by those skilled in the art Routes of administration contemplated by this aspect of the invention include, for example, oral and parenteral administration, with parenteral administration being preferred. Suitable routes for parenteral administration are, for example, the subcutaneous, the intramuscular, and preferably the intravenous route. Modes of administration contemplated by this aspect of the invention include single bolus administration of the compound of Formula I, or continuous infusions, which may extend over extended periods. The length of infusion time may depend on the medical purpose for which infusions are administered, and on the individual subject's needs, as the case may be. Appropriate dose levels for inducing or maintaining a conscious sedated state in a subject by single or repeated bolus injections range from about 2 mg/kg to about 20 mg/kg, preferably from about 2 mg/kg to less than 15 mg/kg, and more preferably from about 5 mg/kg to about 10 mg/kg, The induction of a conscious sedated state through a bolus injection of a compound of Formula I may require a higher dose than the maintenance of an existing conscious sedated state. Thus, for example, a conscious sedated state can be induced in a patient by a bolus injection of about 7.5 mg/kg to about 10 mg/kg, and thereafter maintained by additional bolus injections of about 2 to about 4 mg/kg. If a sedated state is to be induced or maintained by parenteral infusion, appropriate rates of infusion of a compound of Formula I typically range from about 5mg/min to about 25 mg/mm, more typically from about 7 mg/min to about 20 mg/min, and even more typically from about 7 mg/min to about 15 mg/min. As is the case for administration via bolus injections, infusion rates for the induction of conscious sedation may be higher than rates for the maintenance of conscious sedation. Thus, for example, a conscious sedated state can be induced in a patient by infusion of about 7 mg/min to about 25 mg/min, and thereafter maintained by infusion of about 5 mg/min to about 15 mg/min.

Extended periods of conscious sedation are desirable, for example, in procedures that tend to have an unsettling effect on the subject, e.g., imaging studies during which subjects are confined to a narrow NMR tube for extended periods; colonoscopy; surgery under spinal anesthesia; eye surgery; and the like.

The compounds of Formula I possess excellent properties as infusible agents, and their administration by infusion to a subject may be accomplished in several ways, as will be appreciated by those skilled in the art. For example, the compound may be infused via an intravenous "drip." Alternatively, electronically controlled infusion pumps may be employed. Depending on the medical purpose for which infusions are administered, and on the subject's individual needs, the rate of infusion may be adjusted from time to time. A subject in need of sedation, for example, may, during the course of a medical procedure, require increased or decreased rates of infusion to adjust the depth of sedation as needed. A subject in need of anesthesia, for example, may initially receive a compound of Formula I at a bolus dose or at a high infusion rate to achieve rapid loss of consciousness, followed by a lower infusion rate to maintain an unconscious state during a medical procedure; the depth of anesthesia may again be controlled by increasing or decreasing the rate of infusion. For example, to increase the depth of anesthesia in an unconscious subject, the infusion rate may be increased to a rate of up to about 500 mg/min for a short period of time.

Variations in the rate of infusion may be predetermined for a given procedure by using programmable variable-rate infusion pumps. It may thereby be possible to achieve predicted or actual plasma or effect site concentrations of the compound of Formula I- or of propofol derived therefrom " in individual subjects as the medical procedure requires. Variable-rate infusion pumps may also be employed for patient-controlled administration of a compound of Formula I. Such patient-controlled administration may, for example, be useful for conscious patients in need of sedation, for the control of nausea and emesis, such as post-surgical or cancer chemotherapy-related nausea and emesis, and for controlling localized or general intractable itching associated with pruritic conditions.

Those skilled in the art will appreciate that compounds of Formula I, while being useful in the induction and maintenance of anesthesia and sedation as described above, are also useful in treating other medical conditions known to be amenable to treatment with propofol. Therefore, there is provided in another aspect of this invention a method of suppressing nausea or vomiting in a subject, wherein a compound of Formula I is administered to a subject in an amount sufficient to suppress nausea or vomiting. This aspect of the invention has particular applications in settings where the subject suffers from nausea or vomiting related to cancer chemotherapy, or where the subject suffers from postoperative nausea and vomiting. The mode of compound administration within this aspect of the invention may be by single bolus administration, or by constant or variable-rate infusion, as described above. The compounds may be administered via the oral or the parenteral routes, with parenteral and particularly the intravenous route being preferred. Within this aspect of the invention, compounds of Formula I are preferably administered at sub-hypnotic doses, i.e., the plasma concentrations of propofol achieved after administration of a compound of Formula I do not cause loss of consciousness, and, if the subject is not also in need of sedation, preferably do not cause a sedated state. If nausea or vomiting is to be treated by parenteral infusion of a compound of Formula I, for example, appropriate rates of infusion typically range from about 1 mg/min to about 20 mg/min, more typically from about 2 mg/min to about 15 mg/min.

Another aspect of the present invention provides a method of treating itching associated with a pruritic condition in a subject, wherein a compound of Formula I is administered to a subject in an amount sufficient to prevent, alleviate, or suppress localized or general itching. The mode of compound administration within this aspect of the invention may be by single bolus administration, or by constant or variable-rate infusion, as described above. The compounds may be administered via the oral or the parenteral routes, with parenteral and particularly the intravenous route being preferred. Within this aspect of the invention, compounds of Formula I are preferably administered at sub-hypnotic doses, i.e. the plasma concentrations of propofol achieved after administration of a compound of Formula I do not cause loss of consciousness, and, if the subject is not also in need of sedation, preferably do not cause a sedated state. If itching associated with a pruritic condition is to be treated with a parenteral infusion of a compound of Formula I, for example, appropriate rates of infusion typically range from about 1 mg/min to about 20 mg/min, more typically from about 2 mg/min to about 15 mg/min.

The compound of Formula I, or a pharmaceutically acceptable salt thereof, may be administered for treating subjects suffering from an epileptic condition. A subject in need of such treatment is administered a dose of a compound of Formula I in an amount sufficient to prevent, suppress, or alleviate the epileptic condition. This embodiment of the invention finds particular application in the treatment of *status epilepticus.* Suitable dosages for treating patients suffering from an epileptic condition range from sub-hypnotic doses, as defined above, to higher, hypnotic doses, as required by the individual patient's needs. Individual suitable doses can be determined by those skilled in the art, especially in light of the guidance provided herein. A suitable dose for an unconscious patient presenting with *status epilepticus,* for example, may be determined and adjusted as needed by monitoring brain seizure activity on an electroencephalogram, in analogy to the methods employed in Example 1, below. If an epileptic condition is to be treated by single or repeated bolus injections of a compound of Formula I, for example, appropriate doses typically range from about 0.1 mg/kg to 40 mg/kg, more usually from about 1 mg/kg to about 30 mg/kg, and even more usually from about 5 mg/kg to about 20 mg/kg body weight. If an epileptic condition is to be treated by parenteral infusion of a compound of Formula I, for example, appropriate rates of infusion typically range from about 1 mg/min to about 30 mg/min, more typically from about 2 mg/min to about 20 mg/min.

In another aspect, the present invention provides a method for inhibiting oxidation of biological material, wherein the material is contacted with an effective amount of a compound of Formula I.

The present invention also provides a method for the treatment of a pathologic condition having an inflammatory component in a patient, wherein a pharmacologically effective amount of a compound of Formula I is administered to the patient. This embodiment of the invention finds particular application in the treatment of a pathologic condition of the nervous system having an inflammatory component.

In another aspect, the present invention provides a method for the treatment of a pathologic respiratory condition in a patient, wherein a pharmacologically effective amount of a compound of Formula I as defined above is administered to the patient. This embodiment of the invention finds particular application in pathologic respiratory conditions associated with oxidative tissue damage.

In another aspect, the present invention provides a method of treatment wherein a compound of Formula I as defined above is administered to a subject in conjunction with a cytostatic chemotherapeutic agent, and wherein the subject suffers from cancer.

The dosages described above are exemplary and should not be construed as limiting the invention. As will be apparent to persons skilled in the art, many factors that modify the action of the drug will be taken into account in determining the dosage including the age, sex, diet and physical conditions of the subject.

Methods for the chemical synthesis of the propofol prodrug of Formula I from propofol are described in U.S. Patent 6,204,257 to Stella et al., the disclosure of which hereby is incorporated herein by reference. The propofol prodrug of Formulas I is water soluble and can be formulated in aqueous solutions or in other suitable pharmaceutical compositions. The composition may contain an effective amount of the compound of Formula I in combination with one or more pharmaceutically acceptable carriers, excipients, diluents, and/or adjuvants. Any pharmaceutically acceptable aqueous medium may be used to prepare the formulations, such as sterile water, physiological saline, or a mixture of water and an organic solvent, such as propylene glycol, ethanol, and the like. The concentration of the prodrug in the formulation most often ranges from about 0.5 to about 20% (w/v), more usually from about 1 to about 10%.

The formulation also may contain an antioxidant to prevent or reduce oxidative degradation of the prodrug into poorly water-soluble compounds. When present, the concentration of antioxidant most often ranges from about 0.1 to about 1% (w/v). A variety of antioxidants may be used, including without limitation monothioglycerol, glutathione, citric acid, ascorbic acid, sodium metabisulfite, and metal chelators, such as EDTA.

When the composition is formulated for parenteral administration, it is preferable to make up solutions such that the tonicity, *i.e.*, osmolality, is essentially the same as normal physiological fluids in order to prevent post-administration swelling or rapid absorption of the composition because of differential ion concentrations between the composition and physiological fluids. If needed, a tonicity modifier is present in a suitable amount that can be ascertained by persons skilled in the art with the aid of no more than routine experimentation. When used, the amount of tonicity modifier used most often ranges from about 0.1 to about 1% (w/v). Suitable tonicity modifiers include, without limitation, sodium chloride, glycerin, boric acid, calcium chloride, dextrose, and potassium chloride.

The pH of the formulation preferably is maintained to provide long-term stability of the formulation at room temperature. In most cases a suitable pH is from about 7 to about 10, and preferably is at least about 8.5. The solution may be buffered using any standard buffer effective in the pH range of 7-10, *e.g.*, carbonate, phosphate, borate, or glycine. One preferred buffer is tromethamine (2-amino-2-hydroxymethyl-1,3-propanediol), also commonly referred to as TRIS. The amount of buffer needed for this purpose most often ranges from about 10 to about 25 mmol.

Other components may be present in the formulation. For example, in the case of a multi-dose vial, a preservative may be included, such as benzyl alcohol. The formulation also may contain co-solvents such as polyethylene glycol (PEG 200, PEG 400), propylene glycol, and/or ethanol. Concentrations of the co-solvents can vary over a wide range, most often from 0 to about 20%.

The formulations may be packaged, for example, in a glass vial, in a prefilled syringe, or in an ampoule. The formulations may be administered with standard IV diluent solutions, *e.g.*, D5W, normal saline, or Lactated Ringer's solution.

Single bolus doses of the water-soluble prodrug of Formula I were found to cause loss of consciousness with substantially the same rapidity as an equipotent rapid infusion of propofol. It was found that single bolus doses of compounds of Formula I are not associated with pain at the injection site and result in relatively longer durations of unconsciousness. In addition, it was found that the administration of compounds of Formula I is associated with less frequent occurrences of apnea and other side effects conventionally associated with propofol. These factors contribute to the ability to administer appropriate concentrations of compounds of Formula I in single, bolus doses that are effective for inducing anesthesia.

The concentration of the prodrug in the formulation varies depending on the particular treatment for which it is intended and typically ranges from about 0.5 to about 20% (w/v), more usually from about 1 to about 10%. A preferred mode for single bolus dose administration is intravenous injection.

General anesthesia can be induced in a subject by administering a single bolus dose of a compound of Formula I in an amount sufficient to cause loss of consciousness. Similarly, general anesthesia can be maintained in a subject by administering single or repeated bolus doses of a compound of Formula 1. Suitable effective doses for the bolus injection typically range from about 10 to about 50 mg/kg body weight. The lower end of the suitable dose range for general anesthesia will typically be somewhat higher than the doses required for conscious sedation. Thus, while many factors affect an individual patient's response to the action of the drug, as will be appreciated by those skilled in the art, doses used for the induction or maintenance of general anesthesia are preferably higher than 10 mg/kg. Typical preferred ranges are >10 mg/kg to about 40 mg/kg, and more usually from about 15 mg/kg to about 30 mg/kg body weight. The induction of general anesthesia through a bolus injection may require a higher dose than maintenance of anesthesia through subsequent bolus injections. Thus, for example, a bolus dose of about 15 mg/kg to about 30 mg/kg may be used to cause loss of consciousness in a patient, and the general anesthetized state may thereafter be maintained by bolus injections of about 10 mg/kg, to about 20 mg/kg. Many factors may modify the action of the drug and will be taken into account in determining the dosage, including the age, sex, diet and physical conditions of the patient. Those skilled in the art will be able to ascertain, without undue experimentation, appropriate treatment protocols for administering the propofol prodrug. The dosage and schedule of administration are not particularly restricted. Thus, the formulation may be administered via any suitable route of administration, and preferably is administered parenterally. Suitable routes for parenteral administration of a compound of Formula I include, without limitation, the subcutaneous, intramuscular, and preferably the intravenous routes.

The dose of the compound of Formula I for administration by single bolus injection may be adjusted to achieve varying durations of unconsciousness or conscious sedation, as may be required by the medical procedure, or the individual patient's medical needs. For example, if a duration of unconsciousness of about 25 minutes is required, the subject may receive a single intravenous bolus dose of about 20mg/kg of a compound of Formula I. If, for example, a duration of unconsciousness in the range of about 45 minutes is required, the intravenous bolus dose may be increased to about 25 mg/kg. Injection of about 10 mg/kg, on the other hand, can be used to induce a conscious sedated state of about 15 to about 25 minutes in duration. By varying the administered bolus dose of the compound of Formula I, one skilled in the art can thus vary the duration of unconsciousness and/or conscious sedation in a given subject according to the medical procedure for which it is induced. Further variations can be introduced by the co-administration of additional drugs. Thus, for example, if a patient is to be sedated using a bolus dose of 5 - 10 mg/kg of the compound of Formula I, the depth and duration of sedation can be varied by co-administration of opiate analgesics such as meperidine or fentanyl. A suitable dose of fentanyl for co-administration would be about 0.1 to about 3 micrograms/kg body weight, preferably about 0.5 to about 2, and more preferably about 1.5 micrograms/kg.

A single bolus dose of a compound of Formula I is effective for treating nausea or vomiting, particularly post-operative nausea and vomiting (PONV), and nausea and vomiting associated with cancer chemotherapy. To achieve an anti-emetic effect, the prodrug is preferably administered at sub-hypnotic doses, i.e. the plasma concentrations of propofol achieved after administration of the prodrug do not cause loss of consciousness, and, if the subject is not also in need of sedation, preferably do not cause a sedated state. Exemplary doses for a bolus injection of a compound of Formula I to suppress nausea or vomiting range from about 0.1 mg/kg to about 15 mg/kg, preferably from about 1 mg/kg to about 10 mg/kg, and more preferably from about 1 mg/kg to about 5 mg/kg. Formulations for treating nausea or vomiting may be administered in any suitable form and preferably are administered parenterally. Suitable routes for parenteral administration include, without limitation, the subcutaneous, the intramuscular, and preferably the intravenous routes.

A single bolus dose of a compound of Formula I also is effective for treating pruritus. Pharmaceutical compositions for treating pruritus comprise an effective amount of a compound of Formula I sufficient to prevent or suppress pruritus and a pharmaceutically acceptable carrier diluent, or excipient. The compositions may be administered in any suitable form and preferably are administered intravenously. The prodrug is preferably administered at sub-hypnotic doses, i.e. the plasma concentrations of propofol achieved after administration of the prodrug do not cause loss of consciousness, and, if the subject is not also in need of sedation, preferably do not cause a sedated state. Exemplary doses for a bolus injection of a compound of Formula I to treat pruritus range from about 0.1 mg/kg to about 15 mg/kg, preferably from about 1mg/kg to about 10 mg/kg, and more preferably from about 1 mg/kg to about 5 mg/kg. Formulations for treating pruritus may be administered in any suitable form, and preferably are administered parenterally. Suitable exemplary routes for parenteral administration are, without limitation, the subcutaneous, the intramuscular, and preferably the intravenous routes.

The pharmaceutical compositions of the present invention also are effective to cause an anti-emetic effect in a subject. Suitable exemplary anti-emetic compositions contain an effective amount of a compound of Formula I, a second anti-emetic agent, and a pharmaceutically acceptable carrier, diluent, or excipient. Second anti-emetic agents suitable for formulation in the pharmaceutical compositions of the present invention are well-known to those skilled in the art, and include, without limitation, anticholinergic agents, antihistaminergic agents, butyrophenones, phenothiazines, cannabanoids, benzamides, glucocorticoids, benzodiazepines, and serotonergic antagonists. Specific antiemetic agents include, for example, atropine, hyoscine, diphenhydramine, prochlorperazine, chlorpromazine, haloperidol, droperidol, tetrahydrocannabinol, metoclopramide, trimethobenzamide, dexamethasone, lorazepam, and odansetron.

In addition, the pharmaceutical compositions of the present invention are effective to cause an anti-pruritic effect in a subject. Suitable exemplary anti-pruritic compositions contain an effect amount of a compound of Formula I, a second anti-pruritic agent, and a pharmaceutically acceptable carrier, diluent, or excipient. Second anti-pruritic agents suitable for formulations in the pharmaceutical compositions of the present invention are well-known to those skilled in the art, and include, for example antihistamines and corticosteroids.

Other active components may be present in, or may be co-administered with, the formulations of this invention. The additional active components include without limitation hypnotic, analgesic, anti-inflammatory, amnesic, muscle relaxant, and sedative agents. Non-limiting examples include thiopentone, methohexitone, diazepam, midazolam, ketamine, etomidate, propofol, droperidol, morphine, pethidine, fentanyl, meperidine, alfentanil, sufentanil, and remifentanil. Suitable amounts of such these active components can be ascertained by persons skilled in the art with the aid of no more than routine experimentation.

The formulations may be packaged, for example, in a glass vial, in a prefilled syringe, or in an ampoule. The formulations may be administered with standard IV diluent solutions, e.g., D5W, normal saline, or Lactated Ringer's solution.

### EXAMPLES

The following examples are provided to facilitate a better understanding of the invention. The examples should be regarded as illustrative rather than limiting. An aqueous-based 2% solution of O-phosphonooxymethyl-propofol was prepared having the composition set forth in Table 1 below.

**Table 1**

| **Component** | **Concentration** |
|---|---|
| O-phosphonooxymethyl propofol | 2% (20 mg/ml) |
| Sodium Chloride | 0.4% |
| Monothioglycerol | 0.5% |
| TRIS, USP (Tromethamine) | 20 mmol |
| PH | 9±0.5 |

### EXAMPLE 1

This example compares the effects of target controlled infusions (TCI) of propofol and of O'-phosphonooxymethyl propofol disodium salt (AQUAVAN™) on electrical brain activity and consciousness of healthy male volunteers. A sterile solution was prepared in a 20 mL vial, with 20 mg/mL of AQUAVAN™ and 0.4 wt% NaCl. The pH of the solution was adjusted to 8.6 ± 0.4 with HCl or NaOH, as needed.

Nine male volunteers (age 19 to 35 yrs., body wt. 70 to 86 kg) received propofol as a target controlled infusion (TCI) with three different target concentrations over 60 min. After a washout period of 14 days, the same volunteers received a TCI infusion of AQUAVAN™ with identical propofol target concentrations (crossover design). The infusion scheme was a linear increasing propofol concentration up to 5 micrograms/ml for the first 20 minutes, a target of 3 micrograms/ml for the following 20 minutes, and a target propofol plasma concentration of 1.5 micrograms/ml for the following 20 minutes. At 60 minutes, the infusion was stopped.

The EEG was recorded using the CATEEM® system and the data analysis was based on the lead O₁-C_{z}. All subjects were tested every 1.5 minutes for Loss and Recovery of Consciousness (LOC and ROC), and for Loss and Recovery of Corneal Reflex (LOCR and ROCR). Arterial blood samples were drawn at predetermined times up to 240 minutes after start of the infusion. The AQUAVAN™ and propofol plasma concentrations were measured with an LC/MS/MS validated assay and an HPLC-FL validated assay (see Figure 1)

With increasing propofol plasma concentrations, both groups showed a decrease of the Median Power Frequency (MPF) in the EEG, and a shift from an alpha band-dominated EEG to a delta band-dominated EEG. Although comparable plasma concentrations of propofol were achieved in both groups, there was a greater decrease of the MPF for plasma propofol from AQUAVAN™, as compared to plasma propofol from propofol, and this effect lasted longer after the infusions had been stopped.

Clinical parameters also illustrate the effectiveness of infusions of AQUAVAN™: Subjects infused with AQUAVAN™ displayed onset of anesthesia at about the same time after start of infusions as did subjects infused with propofol. The effects of AQUAVAN™ were also longer-lasting than those of propofol, as is shown in Table 2, below:

**Table 2**

| Time (min.) to: | AQUAVAN™ | propofol |
|---|---|---|
| LOC | 9 ± 2 | 13 ± 2 |
| LOCR | 16 ± 5 | 19 ± 6 |
| ROCR | 46 ± 15 | 32 ± 10 |
| ROC | 73 ± 13 | 47 ± 10 |

### EXAMPLE 2

This example demonstrates the effects of target controlled infusions (TCI) of AQUAVAN™ on levels of alertness and sedation of healthy volunteers. A sterile solution of AQUAVAN™ was prepared as described for Example 1, above.

Six female (28 ± 3 yrs, 57 ± 4 kg body weight) and 6 male (32 ± 6 yrs., 78 ± 9 kg body weight) volunteers were studied. For a period of 2 hours, a TCI infusion of AQUAVAN™ was administered to provide adequate sedation. The initially selected target concentration of propofol from AQUAVAN™ was 1.8 µg/ml. Sedation was rated as adequate if according to a modified Observers Assessment of Alertness and Sedation Scale (OAA/S) the OAA/S scale value was 2 to 3. After 60 minutes, the target concentration was increased to 2.4 or 3 µg/ml if the OAA/S scale value was 4 or 5, respectively, or reduced to 1.4 µg/ml if the OAA/S scale value was 0 to 1. With an Aspect^{®} A-1000 monitor and two frontal leads the BIS values were recorded. The ECG, blood pressure, heart rate, SaO₂ and GPI as well as propofol plasma concentrations were measured. All values are given as mean ± standard deviation.

The amount of AQUAVAN™ infused over 2 hours was 2534 ± 506 mg. The OAA/S scale value after 60 minutes was 3.7 ± 1.1. At 60 minutes, target concentration had to be increased to 2.4 µg/ml in 7 volunteers and to 3.0 µg/ml in 2 volunteers. Figure 2 shows the mean results for BIS and OAA/S scale values as well as the measured propofol plasma concentrations. At 4.2 ± 2.5 minutes after the TCI target had been changed, the OAA/S scale value was in the target range of 2 to 3. At 18 ± 3 minutes after stopping the infusion, the volunteers had recovered to an OAA/S scale value of 5. BIS values decreased in parallel with decreasing OAA/S scale values from 96 ± 2 before infusion to 74 ± 13 during the first hour and to 64 ± 14 during the second hour of infusion. The measured propofol plasma concentrations were 1.2 ± 0.39 and 1.9 ± 0.66 µg/ml for the first and second hour of infusion, respectively. The systolic BP decreased from 134 ± 14 to 106 ± 10 mm Hg or 21%. Heart rate increased from 64 ± 14 to 72 ± 8 or 13%.

These results demonstrate that an infusion of AQUAVAN™ can be administered to maintain an adequate level of sedation with an OAA/S scale value of 2 to 3 for 2 hours. With a TCI infusion, an increase of the blood target concentration is rapidly followed by increasing levels of sedation.

### EXAMPLE 3

This example compares the pharmacodynamics of propofol when administered as a single, bolus dose of the prodrug O-phosphonooxymethyl-propofol di-sodium salt (AQUAVAN™) in accordance with the present invention, to the pharmacodynamics of propofol when administered as such. A sterile solution, the "study formulation," was prepared in a 20 mL vial, with 20 mg/mL of AQUAVAN™ and 0.4 wt% NaCl. The pH of the solution was adjusted to 8.6 ± 0.4 with HCl or NaOH, as needed.

Twenty-four healthy subjects (all ASA 1, 25±5 yrs., 70±8 kg) were randomized into 4 cohorts, with 3 male and 3 female in each. Each of the four cohorts was given a single dose of the study formulation described in Table 1 above (5, 10, 20, and 25 mg/kg, AQUAVAN™ respectively). Anesthetic effect was measured continuously using a BIS-XP monitor (Aspect Medical Systems, Natick, MA). The lowest BIS level (BISₚₑₐₖ) was recorded. One week later, propofol was given to the same individuals at a rapid infusion rate of 300 ml/h to reach a similar BISₚₑₐₖ. Heart rate (HR), O₂ saturation, systolic (SBP) and diastolic (DBP) blood pressure were monitored non-invasively. Incidence and duration of apnea, loss (LOC) and return (ROC) of consciousness and duration of unconsciousness (DOU) were measured with the OAA/S score. Adverse events were recorded. Statistical analysis was performed using Wilcoxon Signed Ranks Test, Mann-Whitney-U test, Chi-Square test and Pearson correlation where appropriate.

In the 5 and 10 mg/kg AQUAVAN™ cohorts, no LOC was reached. In 20 and 25 mg/kg AQUAVAN™ cohorts, all subjects reached LOC. There was no significant difference for time to LOC between AQUAVAN™ and propofol. ROC occurred significantly later and DOU was significantly longer for (AQUAVAN™) compared to propofol (Table 3). While the onset and decline in BIS was similar, the AQUAVAN™ BISₚₑₐₖ occurred later than with propofol (AQUAVANT™: 630±225s; propofol: 358±315s, p<0.05). Pain on injection was only reported for propofol, which occurred in 10 of the 24 subjects. Following administration, an initial increase in heart rate (HR>90 bpm) was observed in 17 of the 24 subjects for AQUAVAN™ and in 4 of the 24 subjects for propofol (p<0,05), as well as an increase in SBP and DBP. With AQUAVAN™, early increases in blood pressure were less pronounced with a less clear onset-time. After the initial increase, a similar decrease in SBP and DBP without clinically relevant hypotension was found with both drugs. However, peak decrease occurred earlier with AQUAVAN™ than with propofol. Dose-dependent apnea was more pronounced with propofol (10 of 24 subjects) than with AQUAVAN™ (7 of 24 subjects). BIS and OAA/S were highly correlated for both AQUAVAN™ (r = 0.8937) and propofol (r= 0.7960).

Bolus administration of AQUAVAN™ was shown to achieve LOC at a substantially similar time as an equipotent rapid infusion of propofol, but showed a longer time to peak and slower offset of anesthetic effect. Hemodynamics were similar with both groups, except for an initial tachycardia in AQUAVAN™ during a short-lasting tingling sensation after the injection. Pain on injection and apnea were more pronounced from administering propofol than from administering AQUAVAN™.

**Table 3**

| Cohort | n | Time (seconds) to: | AQUAVAN™ | Propofol |
|---|---|---|---|---|
| Cohort 1 (5 mg/kg) | 6 | | - | - |
| Cohort 2 (10 mg/kg) | 6 | | - | - |
| Cohort 3 (20 mg/kg) | 6 | LOC | 157 ± 69 | 184 ± 63 |
| | | ROC | 1664 ± 920 | 600 ± 105 * |
| | | DOU | 1507 ± 914 | 416 ± 94 * |
| Cohort 4 (25 mg/kg) | 6 | LOC | 162 ± 40 | 208 ± 84 |
| | | ROC | 2865 ± 245 | 956 ± 294 * |
| | | DOU | 2703 ± 267 | 748 ± 245 * |

| | | | | |
|---|---|---|---|---|
| * = p < 0.05 difference between AQUAVAN™ and propofol; mean ± standard deviation. No loss of consciousness (LOC) occurred with bolus doses of 5mg/kg (Cohort 1) and 10 mg/kg (Cohort 2). | | | | |

In addition to induction of unconsciousness and duration of general anesthesia (see above), the response of the study subjects to AQUAVAN™ was also examined with regard to sedation. For this purpose, two additional cohorts (n=6 each) were dosed with 15 and 30 mg/kg of AQUAVAN™ as described above. For subjects in all six cohorts, the lowest OAA/S score achieved after AQUAVAN™ administration and the duration of sedation were recorded, with an OAA/S score of 5 indicating no sedation, scores ranging from 4 - 2 indicating increasingly deep sedated states, and a score of 1 indicating loss of consciousness (LOC)(but allowing for an undirected response to painful tactile stimuli). The findings of this analysis are summarized in Table 4, below. Conscious sedation, but no loss of consciousness, was observed at dose ranges of 5 - 10 mg/kg. Bolus doses of 15 mg/kg and higher induced unconscious states that varied in duration, depending on the dose used. A conscious sedated state typically preceded and followed a period of unconsciousness in subjects who reached LOC. "Mean duration of sedation/LOC" in Table 4 refers to the total time interval from onset of sedation through LOC to recovery of alertness (no sedation).

**Table 4**

| Cohort | Lowest OAA/S Score | Mean duration of sedation /LOC(min) | Duration - range (min) |
|---|---|---|---|
| Cohort 1: 5 mg/kg | 3/6 - 5 (no sedation) | 4.2 | 0-16 |
| | 3/6 - 4 | | |
| Cohort 2: 1 mg/kg | 1/6 - 5 | 14 | 0-24 |
| | 516 - 4 | | |
| Cohort 5: 15 mg/kg | 6/6 - 1 (LOC) | 22.6 | 18-34 |
| Cohort 3: 20 mg/kg | 6/6 - 1 | 39.5 | 22-45 |
| Cohort 4: 25 mg/kg | 6/6 - 1 | 57.8 | 42-78 |
| Cohort 6: 30 mg/kg | 6/6 - 1 | 54.3 | 43-96 |

While particular embodiments of the present invention have been described and illustrated, it should be understood that the invention is not limited thereto since modifications may be made by persons skilled in the art. The present application contemplates any and all modifications that fall within the spirit and scope of the underlying invention disclosed herein.

### Further embodiments

1. A method of inducing or maintaining general anesthesia comprising administering to a subject in need thereof at least one bolus injection of a compound of Formula I: 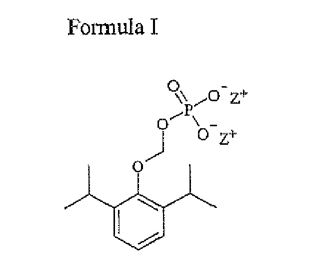 or a pharmaceutically acceptable salt thereof, wherein each Z is independently selected from the group consisting of hydrogen, alkali metal ion, and amine;
   wherein the compound is administered in an amount of from greater than 10 to about 50 mg per kilogram of body weight.
2. The method of item 1 wherein the compound is administered in an amount of from about 15 to about 30 mg per kilogram of body weight to cause loss of consciousness.
3. The method of item 1 wherein the compound is administered in an amount of from greater than 10 to about 20 mg per kilogram of body weight to maintain loss of consciousness.
4. A method of inducing or maintaining general anesthesia comprising administering to a subject in need thereof an effective amount of a compound of Formula I: 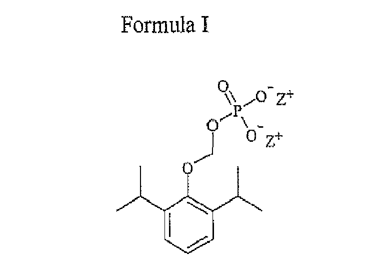 or a pharmaceutically acceptable salt thereof, wherein each Z is independently selected from the group consisting of hydrogen, alkali metal ion, and amine; and
   administering a second anesthetic or sedative agent.
5. The method of item 4 wherein the second anesthetic or sedative agent is selected from the group consisting of midazolam, fentanyl, meperidine, propofol, and combinations thereof.
6. The method of item 5 wherein the second anesthetic or sedative agent is an opiate analgesic selected from the group consisting of meperidine, fentanyl, and combinations thereof.
7. The method of item 4 wherein the compound of Formula I is administered by parenteral infusion.
8. The method of item 4 wherein the compound of Formula I is administered by one or more bolus injections.
9. A method of inducing and maintaining general anesthesia in a subject comprising administering to a subject in need thereof a compound of Formula I: 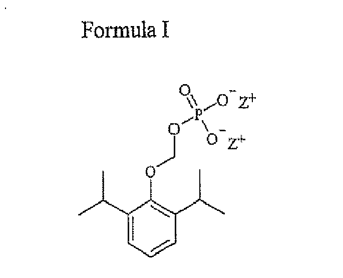 or a pharmaceutically acceptable salt thereof, wherein each Z is independently selected from the group consisting of hydrogen, alkali metal ion, and amine, in a first amount sufficient to cause loss of consciousness; and
   administering, once or repeatedly, to said subject a compound of Formula I, or a pharmaceutically acceptable salt thereof, in a second amount sufficient to maintain loss of consciousness.
10. The method of item 9 wherein the first amount is administered by a bolus injection at a dose of from about 15 to about 30 mg per kilogram of body weight to cause loss of consciousness, and the second amount is administered by a bolus injection at a dose of from about 10 to about 20 mg per kilogram of body weight to maintain loss of consciousness.
11. The method of item 9 wherein the first amount is administered by parenteral infusion, and the second amount is administered by parenteral infusion at a rate of from about 10 to about 35 mg/min to maintain loss of consciousness.
12. A method of producing a sedated state in a subject comprising administering to a subject in need thereof a compound of Formula I: 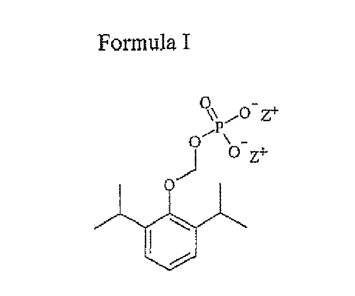 or a pharmaceutically acceptable salt thereof, wherein each Z is independently selected from the group consisting of hydrogen, alkali metal ion, and amine;
   wherein the compound is administered by at least one parenteral bolus injection in an amount of from about 2 mg/kg to less than 15 mg/kg.
13. The method of item 12, wherein the compound is administered in an amount of from about 5 mg/kg to about 10 mg/kg.
14. A method of inducing and maintaining a sedated state in a subject comprising administering to a subject in need thereof a compound of Formula I: 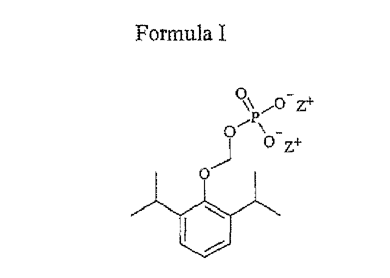 or a pharmaceutically acceptable salt thereof, wherein each Z is independently selected from the group consisting of hydrogen, alkali metal ion, and amine, in an amount sufficient to maintain the sedated state; and
   administering, once or repeatedly, to said subject a compound of Formula I, or a pharmaceutically acceptable salt thereof, in a second amount sufficient to maintain the sedated state.
15. The method of item 14 wherein the first amount is administered by a bolus injection at a dose of from about 5 to about 15 mg per kilogram of body weight, and the second amount is administered by a bolus injection at a dose of from about 2 to about 10 mg per kilogram of body weight.
16. The method item 14 wherein the first amount is administered by a parenteral infusion at a rate of from about 5 mg/min. to about 25 mg/min., and the second amount is administered by a parenteral infusion at a rate of from about 5 to about 15 mg/min.
17. A method of producing a sedated state in a subject comprising administering to a subject in need thereof a parenteral infusion of a compound of Formula I: 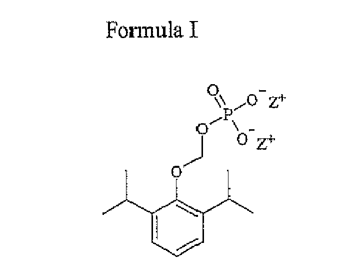 or a pharmaceutically acceptable salt thereof, wherein each Z is independently selected from the group consisting of hydrogen, alkali metal ion, and amine;
   wherein the compound is administered in an amount of from about 5 to about 25 mg/min.
18. The method of item 17 wherein the compound is administered in an amount of from about 7 to about 20 mg/min.
19. The method item 18 wherein the compound is administered in an amount of from about 7 to about 15 mg/min.
20. A method of producing a sedated state in a subject comprising administering to a subject in need thereof an effective amount of a compound of Formula I: 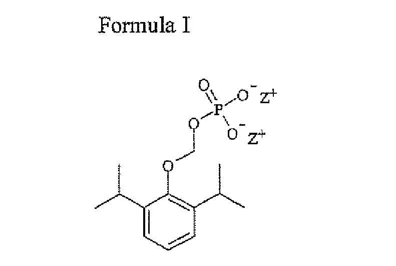 or a pharmaceutically acceptable salt thereof, wherein each Z is independently selected from the group consisting of hydrogen, alkali metal ion, and amine; and
   administering a second agent selected from anesthetic, analgesic, and sedative agents.
21. The method of item 20 wherein the second agent is selected from the group consisting of midazolam, opiate analgesics, propofol, and combinations thereof.
22. The method of item 21 wherein the second agent is an opiate analgesic selected from the group consisting of meperidine, fentanyl, and combinations thereof.
23. The method of item 20 wherein the compound of Formula I is administered by parenteral infusion.
24. The method of item 20 wherein the compound of Formula I is administered by one or more bolus injections.
25. A method of treating at least one condition selected from the group consisting of an epileptic condition, nausea or vomiting, pruritus, pathologic respiratory conditions related to oxidative tissue damage and pathologic conditions having an inflammatory component, the method comprising administering to a subject in need thereof an effective amount of a compound of Formula I: 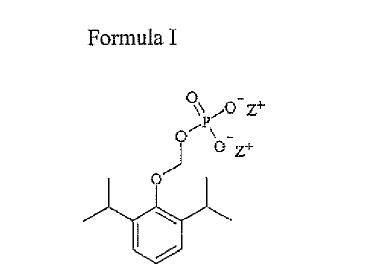 or a pharmaceutically acceptable salt thereof, wherein each Z is independently selected from the group consisting of hydrogen, alkali metal ion, and amine.
26. The method of item 25 wherein the compound is administered by parenteral infusion.
27. The method of item 25 wherein the compound is administered by one or more bolus injections.
28. A pharmaceutical composition comprising an anesthetic effective a compound of Formula I: 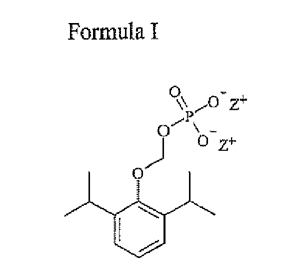 or a pharmaceutically acceptable salt thereof, wherein each Z is independently selected from the group consisting of hydrogen, alkali metal ion, and amine;
   a second anesthetic or sedative agent; and
   a pharmaceutically acceptable carrier, diluent, or excipient.
29. The composition of item 28 wherein the second anesthetic or sedative agent is selected from the group consisting of midazolam, fentanyl, meperidine, propofol, and combinations thereof.
30. The composition of item 29 wherein the second anesthetic or sedative agent is an opiate analgesic selected from the group consisting of meperidine, fentanyl, and combinations thereof.
31. A pharmaceutical composition comprising an anti-emetic effective amount of a compound of Formula I: 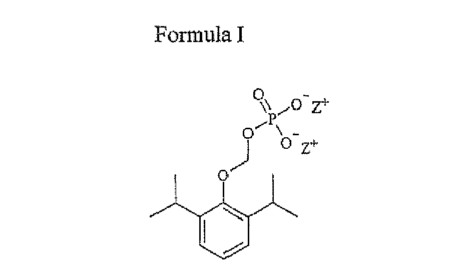 or a pharmaceutically acceptable salt thereof, wherein each Z is independently selected from the group consisting of hydrogen, alkali metal ion, and amine;
   a second anti-emetic agent; and
   a pharmaceutically acceptable carrier, diluent, or excipient.
32. The pharmaceutical composition of item 31 wherein the second anti-emetic agent is selected from the group consisting of anticholinergic agents, antihistaminergic agents, butyrophenones, phenothiazines, cannabinoids, benzamides, glucocorticoids, benzodiazepines, serotonergic antagonists, and combinations thereof.
33. The pharmaceutical composition of item 32 wherein the second anti-emetic agent is selected from the group consisting of atropine, hyoscine, diphenhydramine, prochlorperazine, chlorpromazine, haloperidol, droperidol, tetrahydrocannabinol, metoclopramide, trimethobenzamide, dexamethasone, lorazepam, odansetron, and combinations thereof.
34. A pharmaceutical composition comprising an anti-pruritic effective amount of a compound of Formula I: 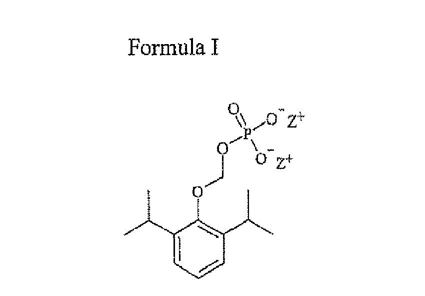 or a pharmaceutically acceptable salt thereof, wherein each Z is independently selected from the group consisting of hydrogen, alkali metal ion, and amine;
   a second anti-pruritic agent; and
   a pharmaceutically acceptable carrier, diluent, or excipient.
35. The pharmaceutical composition of item 34 wherein the second anti-pruritic agent is selected from the group consisting of antihistamines, corticosteroids, and combinations thereof.

## Claims

1. A compound of Formula I for use in a method for producing a conscious sedated state comprising administering to a subject in need thereof at least one bolus injection of a compound of formula I: 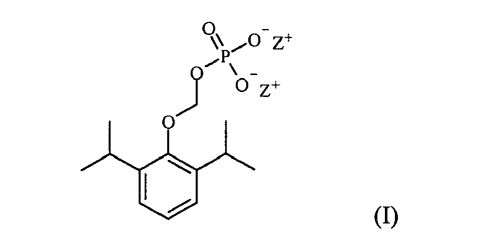 or a pharmaceutically acceptable salt thereof, wherein each Z is independently selected from the group consisting of hydrogen, alkali metal ion and amine;
wherein the compound is administered by at least one parenteral bolus injection in an amount from about 2 mg/kg to less than 15 mg/kg.

2. The compound for use according to claim 1, wherein the compound is administered in an amount of from about 5 mg/kg to about 10 mg/kg.

3. A compound of Formula I for use in a method for inducing and maintaining a conscious sedated state comprising administering to a subject in need thereof a compound of Formula I: 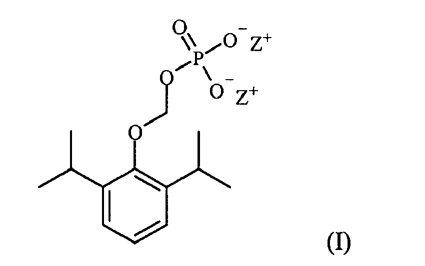 or a pharmaceutically acceptable salt thereof, wherein each Z is independently selected from the group consisting of hydrogen and alkali metal ion,
wherein the compound of Formula I is administered in a first amount by a first bolus injection; and
administering, once or repeatedly, a compound of Formula I or a pharmaceutically acceptable salt thereof, in a second amount sufficient to maintain the conscious sedated state.

4. The compound for use according to claim 3, wherein the first amount is administered by a bolus injection at a dose of from about 5 to about 15 mg per kilogram of body weight and the second amount is administered by a bolus injection at a dose of from about 2 to about 10 mg per kilogram of body weight.

5. The compound for use according to claim 3, wherein the first amount is administered by a parenteral infusion at a rate of from about 5 mg/min to about 25 mg/min, and the second amount is administered by a parenteral infusion at a rate of from about 5 to about 15 mg/min.

6. A compound of Formula I for use in a method for producing a conscious sedated state comprising administering to a subject in need thereof a parenteral infusion of a compound of formula I: 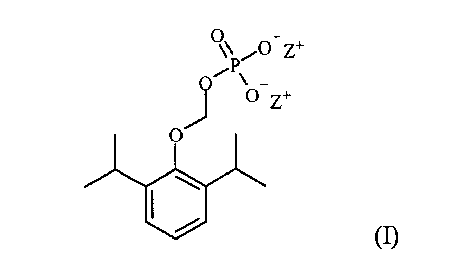 or a pharmaceutically acceptable salt thereof, wherein each Z is independently selected from the group consisting of hydrogen, alkali metal ion and amine;
wherein the compound is administered in an amount of from about 5 to about 25 mg/min to produce the conscious sedated state.

7. The compound for use according to claim 6, wherein the compound is administered in an amount of from about 7 to about 20 mg/min.

8. The compound for use according to claim 6, wherein the compound is administered in an amount of from about 7 to about 15 mg/min.

9. The compound of Formula I for use in a method for producing a conscious sedated state comprising administering to a subject in need thereof at least one bolus injection of a compound of formula I: 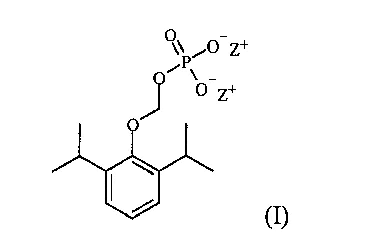 or a pharmaceutically acceptable salt thereof, wherein each Z is independently selected from the group consisting of hydrogen, alkali metal ion and amine;
wherein the compound is administered by at least one parenteral bolus injection in an amount from about 5 mg/kg to about 10 mg/kg.

10. The compound for use according to claim 9, wherein the compound is administered in an amount of from about 5 mg/kg to about 7.5 mg/kg.

11. The compound for use according to claim 10, wherein the compound is O-phosphonooxymethyl propofol disodium salt.
